(19) **Europäisches Patentamt / European Patent Office / Office européen des brevets**

(11) **EP 2 510 976 A1**

(12) **EUROPEAN PATENT APPLICATION**

(43) Date of publication:
**17.10.2012 Bulletin 2012/42**

(51) Int Cl.:
**A61N 5/06** $^{(2006.01)}$

(21) Application number: **11382110.2**

(22) Date of filing: **14.04.2011**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Gomez de Diego, Eduardo Antonio**
**28231 Las Rozas (Madrid) (ES)**

(72) Inventor: **Gomez de Diego, Eduardo Antonio**
**28231 Las Rozas (Madrid) (ES)**

(74) Representative: **Temino Ceniceros, Ignacio**
**Abril Abogados**
**Amador de los Rios 1-1°**
**28010 Madrid (ES)**

(54) **Device for hair growth stimulation**

(57) The present invention relates to a device for stimulating hair growth comprising an outer casing (1) with a first hold end (2) of the device, a second stimulating end (3) and start-up means (6). At the second stimulating end (3) are located at least one laser lighting element (4) and at least one LED lighting element (5). Said laser lighting elements (4) emit a wavelength between 600 and 900 nm, and LED lighting elements (5) a wavelength between 400 and 600 nm. Thus, through laser lighting elements (4) is achieved stimulating the hair regeneration

FIG. 1

## Description

The present invention pertains to the field of devices designed to stimulate hair growth. Specifically, the present invention uses laser diodes and LEDs emitting at wavelengths between 655 and 470 nm respectively, stimulating the hemoglobin, and therefore the blood circulation, and decreasing the sebaceous secretion. The combined effect of the above allows stimulating the hair growth.

STATE OF THE PRIOR ART

The scientific basis underlying the use of the device consists of the interaction (absorption, transmission and scattering) of low power laser light with the different human body tissues and components.

Specifically, its operation falls within the low power laser therapies, LLLT (Low level laser therapy), known since the middle of the XX century and are being used in Europe since 30 years ago to stimulate the vascularization and cell regeneration in animals and humans.

The suitability of using laser light to treat hair loss has been confirmed by numerous scientific studies. The suitability of using laser light to treat hair loss has been confirmed by numerous scientific studies. There are also a good number of studies which, although not find evidence of this operation, indicate that it produces no harmful or negative effect, in fact, normally state that has no effect. However, mostly of these references do not clearly describe the technical specifications of the laser used and the doses deposited on tissues that are so low that it can not be registered any improvement.

Other studies, however, have shown that the use of coherent light - monochromatic laser of certain wavelengths, 630-685 nm and with a dose of between 3 to 4 $J/cm^2$, stimulate both the increase of hair growth as the density of hair follicles.

The hair follicle is the part of the skin that gives the hair growth since has stem cells. Within the follicles are sebaceous glands, destined specifically for the production of sebum that lubricates the hair surface. The thicker the hair, the more sebaceous glands are found.

At the base of the hair, a fine network of blood vessels forms the root thereof. Around of this, it is a white structure called the bulb, visible when healthy hairs are pulled up.

At the base of the follicle there is a small structure called the papilla. Here is where are originated the cells that are part of the hair. The papilla is cone shaped and protrudes into the bulb of growing hair.

Jointly to the follicle there is a tiny muscle group that is responsible for the perpendicularity of the hair to the surface of the skin. Cells located between the erector muscle and the follicle, are essential in hair growth.

Initially it was believed that body hair is produced by rapidly dividing stem cells of the matrix located in the deepest part of the hair follicle, 2 to 7 mm under the skin surface. However, recent evidence suggests that follicular stem cells are located in the outermost layer of the root, in an area near the insertion of hair erector muscle, about 1.5 mm under the epidermis. Both the area of the erector muscle and the bulb are important in hair regeneration and growth.

Some biological reasons why the application of laser light on the area, in appropriate doses, contributes actively to the growth and increase of the thickness of hair. Those reasons are listed below.

First, there is an increase on the blood flow of the scalp at approximately 54%, thus stimulating the transport of nutrients through the blood in the blood vessels surrounding the hair follicle bulb, the transport of nutrients to the cells located between the erector muscle and the follicle, which are essential in hair growth, and transport of toxins and residues from the tissues through the bloodstream.

In the same way, sebum secretion is increased in the early stages of treatment. The size and number of sebaceous glands of a hair follicle is related to the thickness of the hair. By applying light to these areas, an initial increase in sebum production which is very positive is produced, since it reaffirms the thickness of hair. As treatment is applied, the secretion of lipids is becoming normalized.

Additionally, cellular activity of tissue irradiated is increased, a phenomenon known as photobiostimulation. The laser light penetrates the soft tissue, is absorbed and used by the cells that formed it to help in its chemical processes, increasing the action of the molecule adenosine triphosphate, ATP, responsible for transporting energy from cellular reactions. These same regenerative effects, in other type of injuries, severe trauma of the central nervous system, as well as inverse effects on cancer cells, reducing metabolic activity and thus, reducing their growth.

It has also observed an increase in the health of cells irradiated tissue. Although further studies are needed, current research indicates that the changes induced by low power laser lighting resulting from laser-generated production of hydrogen peroxide, a natural byproduct of cellular respiration.

Finally, it has observed a reduction in the amount of bacteria that may be found in the tissue, leaving the skin on the scalp healthier and with less risk of infection and pimples. With regard to the appropriate wavelengths to promote growth and thickness of hair, wavelengths located between 600 and 900 nm are the best to stimulate blood circulation, since they are essentially absorbed by hemoglobin, but also by melanin. Since they use low-power lasers, there is no real danger of stimulating melanin and producing skin spots.

For other wavelengths, there is a light scatter, which is not recommended, and the water contained in tissues can also absorb energy, producing heat, a fact which is not recommended either.

The wavelengths more used for hair growth stimulation are reduced to a range between 630 nm and 685 nm, which is the safest area and where the energy absorption by hemoglobin, to increase blood circulation, is optimal.

In the event that the user does not follow the instructions and administer himself/herself a higher dose than recommended, there is the so-called "bio-suppressive effect". In healthy tissue, very high doses do not affect them. In tissue under treatment, especially in the treatment of wounds and hair loss, the extra dose result in tissue repair take longer than recommended, since paralyzes the cell regeneration and its growth, but results in an increase in immunological activity, which in some cases is appropriate. The bio-suppressive effect is associated with immune-stimulation discussed for example in WO 01/39835.

US 2002/0128696 describes a device to stimulate hair growth contains a low power laser and a perforated mirror to distribute the energy at different points along the device. This system has a big loss of power due to reflections from the mirror, which implies that the minimum dose required for proper operation of the therapy is not reached.

WO 2005/016454 describes an apparatus for healing alopecia comprising a GA-As semiconductor laser with a wavelength between 790 and 904 nm in the near infrared band, optical for the laser, various LED with wavelengths between 630 and 660 nm in the red band, several mirrors of chrome for LEDs and a vibrator motor. It also has several teeth in soft resin to massage the scalp. The use of LEDs in the red band fails to reduce oil secretion, or treat in the surface area of skin, acne-causing bacteria on the scalp or stimulate the surface circulation. It does not describe the use of a contact sensor to ensure that the device is safe (safety group 4 in the use of the laser).

EP2140911 describes the use of, at least, two LEDs emitting at different wavelengths. However, it uses a group of LED in red and, also, does not describe the use of a contact sensor to ensure that the device is safe (safety group 4 in the use of the laser).

EXPLANATION OF THE INVENTION

The invention relates to a device for stimulating hair growth comprising: (a) a first end intended to act as a hold element of the device; (b) a second end in which are the elements intended for stimulating the scalp; and comprising at least one laser lighting element and at least one LED lighting element as well as start up and shutdown means of the device, for example a switch. The start up of the device, due to security issues, will include the turn on of LED lighting elements and the connection of the laser lighting elements, but not its lighting.

According to the invention, the aforementioned laser lighting element emits at a wavelength between 600 and 900 nm, and the said LED lighting element emits at a wavelength between 400 and 600 nm.

Preferably, the emission band of the laser lighting elements will be between 630 and 685 nm, preferably being selected a wavelength of 655 nm. In the case of the emission band of LED lighting elements, will be between 450 and 495 nm, preferably using a wavelength of 470 nm.

The device comprises means for turning on the laser lighting elements when the second stimulating end is in contact with the skin of a user. The device must be turned on by the start up and shutdown means, discussed above, but nevertheless, laser lighting elements will not turn on until the second stimulating end contacts with user skin or scalp. Thus, it is possible to reduce the risk involved with laser lighting elements. Without the presence of these means, laser lighting elements would come into operation when the device starts up, since the laser could focus on the retina and can be a source of risk. Since the operation is limited when comes into contact with the user skin, and not in other circumstances, the risk that the device represents is canceled and can be classified as safe in all reasonably foreseeable conditions.

One of the possible means to turn on the lighting elements is for example conductivity sensors, so that, when detecting conductivity with approximate value to the human skin, the laser lighting elements are turned on. A typical value of resistivity, inverse of the conductivity, of human skin is $5 \times 10^5 \ \Omega$ m.

The device may comprise a vibrator element. Thus, the device can perform actions such as scalp massage, enhancing the effects of the laser and LED lighting elements. The vibrator element will have start up and shutdown means independent of start up and shutdown means of the device. Therefore, the vibrator element only works if the user wants and starts up the element.

In order to act as a comb or brush, the device may include a plurality of protrusions regularly arranged. These protrusions act like the teeth of a comb. The number of such protrusions can be adapted to the desired setting for the device.

The power of each laser lighting element may be within a range between 3 and 10 mW, being preferably a power of 5 mW.

The number of lighting elements may be greater than two, so that the effect of element due to the laser lighting elements will be multiplied by the number of laser lighting elements composed by this. Laser lighting elements may be placed homogeneously distributed on the second stimulating end. When they are distributed homogeneously will not be possible that two laser lighting elements illuminate or have effect at the same time on the same portion of skin or scalp.

Laser lighting elements may be InGaAlP diode lasers.

The start up and shutdown means of the device and the start up and shutdown means of the vibrator may comprise an electrode, a detection chip connected to the electrode through a resistor, a capacitor connected to the detection chip, and a logic interpretation, so that the contact of a part of the user body to the electrode causes a change of state in the start up and shutdown means, so that the device is turned on when it is turned off or vice versa.

Thus, against the cited state of the art, the present invention is novel and inventive as it provides the use of a single LED in the blue range combined with a laser emitting element that only turns on when is in close contact with skin.

Throughout the description and claims the word "comprises" and its variations are not intended to exclude other technical features, additives, components or steps. For those skilled in the art, other objects, advantages and features of the invention will emerge in part from the description and in part from the practice of the invention. The following examples and drawings are provided by way of illustration and not intended to be limiting of the present invention. Furthermore, the present invention covers all possible combinations of particular and preferred embodiments set forth herein.

BRIEF DESCRIPTION OF THE DRAWINGS

In order to complement the description to be made immediately after, and with the object of assisting in a better understanding of the features of the invention, according to a preferred embodiment thereof, is attached as an integral part of this description, a set of drawings wherein with illustrative and non-limitative character, is shown as follows:

FIG 1. Shows a perspective view of the device for hair growth stimulation of the present invention.

FIG 2. Shows a front view of device in Figure 1, and wherein the FIG. 2A shows a section view of the device according to the plane AA' defined in Figure 2.

FIG 3. Shows a rear view of the device in Figure 1.

FIG 4. Shows the electrical diagram of the two start up and shutdown means which comprises the invention.

DETAILED DESCRIPTION OF AN EMBODIMENT

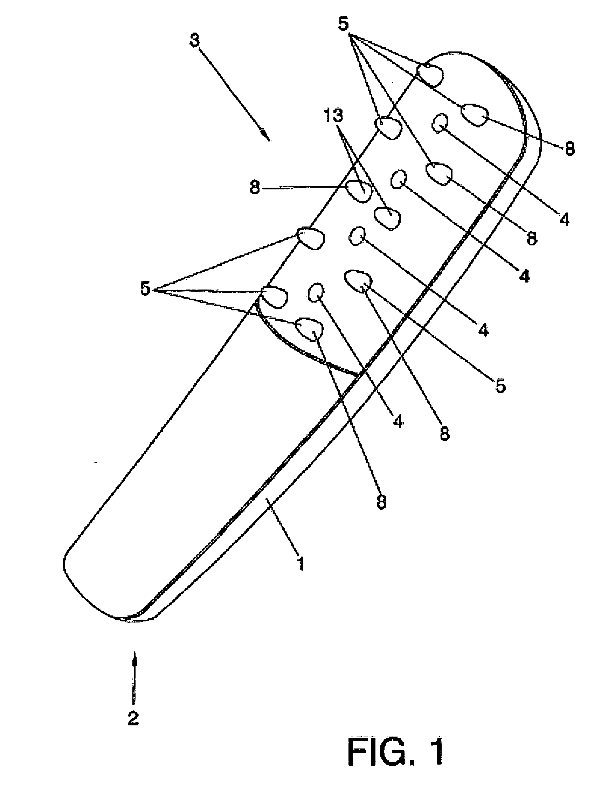

Figure 1 shows the general appearance of the device, it can be observed its two distinct areas, a first hold end (2) and a second stimulating end (3) in which can be observed the protrusions (8) that act like teeth of a brush, with the idea of allowing access to the ends of the protrusions (8) to the user scalp or skin.

The number of protrusions (8) in the present embodiment is ten. The second stimulating end (3), on the side corresponding to the protrusions (8) is made of transparent methacrylate.

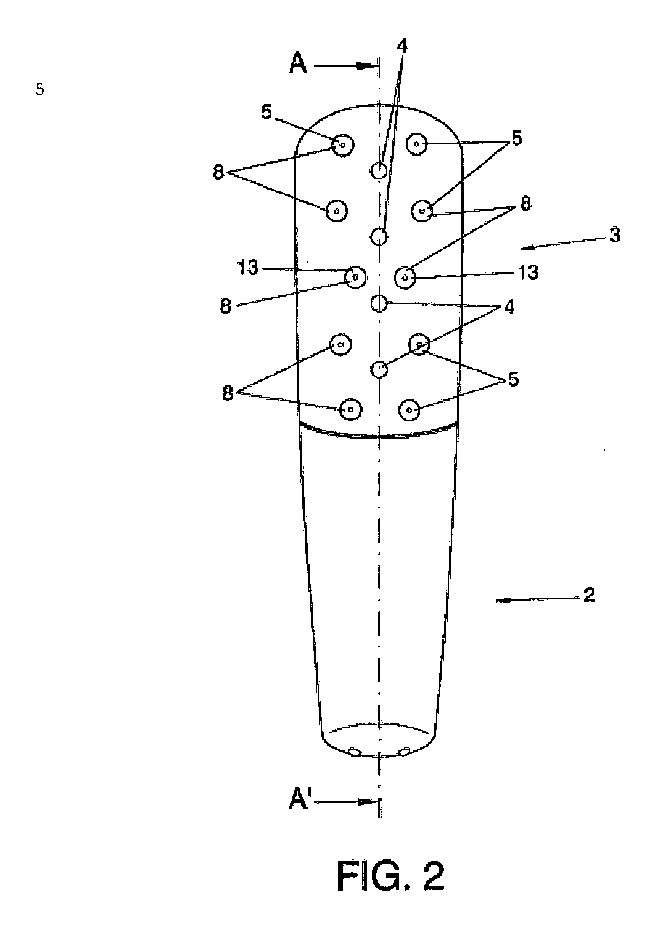

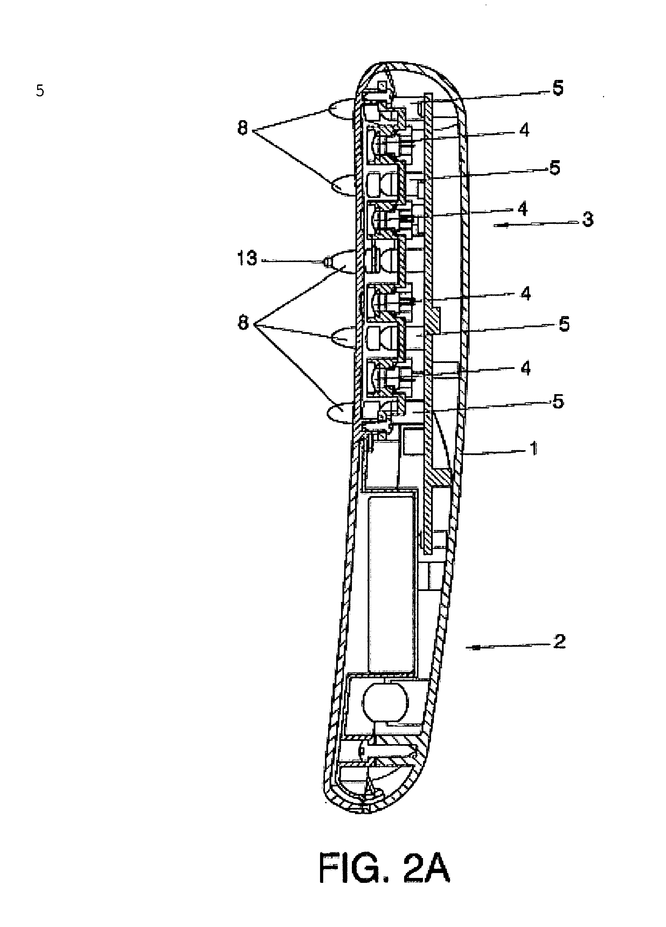

In the second stimulating element (3) are located four laser lighting elements (4) aligned along a longitudinal axis of said element in the middle of the second stimulating element, as shown in Figures 1 and 2. The four laser lighting elements (4) are located inside the second laser lighting element (3). As already mentioned, the casing of said element will be made in transparent methacrylate. In order to define the window through which laser lighting elements (4) will emit, the inside of the second stimulating element (3) will be painted, except in areas in which are defined the necessary windows for the laser lighting elements (4). Thus, the laser light may be emitted without interference and the laser lighting elements (4) protected by the casing of the second stimulating element (3).

The selected laser lighting elements (4) are low power InGaAlP diode laser, 5 mW, at the red spectrum, emitting with a wavelength of 655 nm on the scalp. Laser lighting elements (4) do not have a reflective optics, for example mirrors, to direct the laser light beams, or refractive, for example lenses, to concentrate it. Each laser lighting element includes a transparent plastic window of 1 mm thick, designed to protect the laser lighting elements (4) against dirt.

The fact of not using any type of optics, like for example lenses, mirrors or fiber optic, prevents absorption, reflection and scatter losses at surfaces, and enables the laser diodes are closer to the scalp, making use of all output power of lasers for the treatment.

Powers or doses commonly used for this type of lasers depend on its application. Below are some examples:

- Dental applications: power = 3-10 mW
- Around an open wound: dose = 1-4 $J/cm^2$
- Center of an open wound: dose = 0.5 $J/cm^2$

In the case of scalp stimulation for hair growth and the increase of the density of hair follicles, the doses required are 3-4 $J/cm^2$ in the range of wavelengths of 630-685 nm.

In the present embodiment has been selected a InGaAlP continuous laser by *Roithner Lasertechnik* with a power of 5 mW and wavelength 655 nm, the output beam having a certain divergence in the perpendicular and parallel directions which make the illuminated area is not precise but a small area. According to technical specifications of the product, the parallel and perpendicular divergences of the beam operating at 5 mW of power are respectively 10° and 30°, thus, the illuminated area will be an ellipse.

Since the focus of the lighting elements are placed in the cover of the casing (1) in the second stimulating end (3), and the length of the protrusions (8) defines the separation between the second stimulating end (3) and the scalp, the area covered by each lighting element is defined by that length. In the present embodiment, the length of the protrusions (8) is 10.19 mm.

To calculate the time of application in each area, the area of this ellipsoidal lighting area, the dose required and the continuous laser power must be taken into account. The relation between dose, D, the power of the laser lighting element (4), P, the illuminated area, A, and time, t, is as follows:

$$D = \frac{P \cdot t}{A}$$

The actual values to be used in the present embodiment are:

- Dose, D, 5 J/cm$^2$, considering with this value the possible loss of power, for example by reflection of light by the hair or the distance of application.
- The illuminated area is equal to the area of the ellipse defined by the half-axes that are created by opening the beam of light, according to its parallel and perpendicular divergences over 10.19 mm. Thus, the half minor axis, a, is equal to 0.8881 mm and the half major axis, b, equal to 2.6374 mm, and the area of the ellipse equal to π.ab, equal to 0.0735 cm$^2$.
- The power of laser lighting element (4), P, is equal to 0.005 W.

Thus, the required lighting time of each specific area, each ellipse, is 77 seconds or 1.28 minutes per each zone. Assuming that the device will be used for an average of 15 minutes combing the head, it seems reasonable to expect that passes through each zone for 1.28 min for 15 minutes of combed.

It is appropriated to consider in this discussion that the device has, as already mentioned, four laser lighting elements (4), being the separation between each superior to 12 mm, so its elliptical lighting zones will not overlap treating different areas of the scalp in a same pass of the device.

Depending on the length of time the device is applied to the scalp, the area effectively treated in each session will be higher or lower. In order to achieve an optimal performance of the device, the user must concentrate their use in a limited area for a period of approximately 1.28 minutes discussed above, thus making use not only of the benefits from exposure to laser light and LED diodes, but also of the massage created by the vibration of the device. After the defined time period, it may be treated a second area. Thus, the treatment of the entire scalp will carry out in stages, and may be necessary more than one session for complete treatment.

The laser chosen is a semiconductor laser, so that does not require any time prior to stabilization, but, it is connected its maximum output power is immediately reached making that the device is operating at the same time of the turn-on. It would be otherwise if the laser chosen had been of gas, like for example a HeNe laser.

The depth that reaches a laser like that used in the present embodiment, with a power of 3.5 mW, is 6-8 mm. These penetration values depend on the type of treatment technique used, type of tissue on which they are used and the technical design of the device. With regard to the type of treatment technique used, there is no a limit in the penetration of the laser beam, can go through bone, muscle and fat tissue, the latter being the most transparent of the three.

Given that, according to the evidences of the form of hair growth, the stimulation of the erector muscle area, located at 1.5 mm from the epidermis, and bulb, located at a distance of between 2 and 7 mm under the epidermis, is produced with a laser with a power of 3.5 mW, therefore, the use of four laser of 5 mW has a penetration depth sufficient for effective treatment of the scalp.

Other designs use more powerful lasers with the idea that they have greater penetration depth. This is true, but not necessarily suitable for the purpose of the device as the key of the optimum performance of LLLT in the hair growth stimulation is based on an appropriate dose, not the more power the better. In fact, the same type of lasers can be used for removing the same hair that we intend to make grow only increasing its power. Also, the penetration depth required is that it reaches to the area of the erector muscles and bulb, a greater penetration will increase the cost of the laser, due to the need of more power, without increasing the treatment improvements. For this reason, it is not advisable to use high powers, but adequate doses to the same wavelengths.

The second stimulating end (3) further comprises LED lighting elements (5), located under the area in which the eight end protrusions (8) are located, that is, the group of four protrusions (8) close to end of the device and the group of four protrusions (8) close to the middle of the casing (1), emitting at a wavelength of 470 nm. The two protrusions (8) located in the middle of the area defined by the whole of the protrusions (8) will not cover any LED lighting element (5). Like in the case of windows designed to emit the laser light of the laser lighting elements (4), the inside of the casing (1) of the second stimulating end (3) corresponding to the protrusions (8) mentioned above will not be painted either, so that the protrusions (8) will define the windows through which the LED light will be emitted.

Figure 2A shows the arrangement of lasers and LED diodes inside the device, the first ones placed in the longitudinal axis of the device and the second ones under the protrusions (8).

Numerous studies have shown the suitability of the use of low wavelengths in the treatment of acne in the blue visible range, in the vicinity of 470 nm, and that acne-causing bacteria are focused at those wavelengths in the surface area of the skin.

Since the device has LED lighting elements (5), which provide a slightly penetrating light at 470 nm, can improve other disorders of the skin, reducing fat levels, the size of the pores, the folliculitis, etc. The light intensity, irradiance of the LED, 4500 millicandelas, is superior to any LED used in the market for a similar purpose and its opening angle, 40°, leads to a circular beam of diameter 8 mm with Gaussian profile in the area of the scalp.

According to the quality standard UNE-EN60825-1, the classification of laser equipment depends primarily on the safety in use, with the classification established as follows:

- Class 1: Safe in all reasonably foreseeable conditions.
- Class 2: Low Power, eye protection is usually achieved with defense reflex reactions.
- Class 3: As Class 2. The direct vision in the beam with optical instruments can be dangerous.
- Class 3B: The direct vision in the beam can be dangerous.
- Class 4: High power, the diffuse reflection can be dangerous.

The four lasers used in the present embodiment, InGaAIP semiconductor lasers with a power of 5 rnW at 655 nm, are Class 4 if it does not include any element that makes safe its operation in all reasonably foreseeable conditions.

There are two alternatives to use a Class 4 laser in class 1 equipment:

- Including a contact sensor (13) which activates the laser operation when the equipment detects that it is positioned on the skin/scalp. This system does not alter the specifications of laser light, spatial and temporal coherence, opening, power, but only when it is applied.

- Placing a diffuser in front of the laser beam output, thus breaking the spatial coherence of the laser and its riskiness is eliminated. This system alters the laser specifications since modifies the spatial coherence of laser beam, thus reducing its penetration into the tissues, being necessary then to increase base power laser, increasing the cost of the system. Additionally, the opening of the laser beam is altered making a larger diameter, so that also the effective dose of the tissues is reduced, being necessary to use a more powerful laser. Finally, this modifies the output beam, since being a refractive element, the light must to pass through the diffuser, which always absorbs and reflects part of incident light.

In the present embodiment has been chosen to use a contact sensor (13) that recognizes when the device is in safe operating position, by calibrating the sensor to detect skin/scalp. Thus, the device becomes class 1, and may be distributed as commonly used in the home.

The contact sensor (13) used is a conductivity or resistive sensor due to its ease of implementation, reliability in use and economy. It is based on measuring the resistivity or its inverse, the conductivity, of the medium in contact with the sensor. Said contact sensor (13) will be placed in the device in the two central protrusions (8), that is, those that do not include LED lighting elements (5) beneath it.

The resistive sensor must be calibrated to be able to differentiate between the skin and other materials, like for example the glasses glass. The typical value of resistivity of human skin at 23°C is $5.0 \times 10^5$. The protuberances (8) of the stimulating end are used to separate the hair from the head of the area to be treated, since hair act as a screen, that is, reflects the light, for passing the treatment laser light, preventing the scalp absorbs the energy.

The vibrator element helps to improve the blood circulation in the area to be treated and helps to the absorption of any lotion that the user may use for treating his/her hair. For this reason, it may be convenient to use the device after the hair wash.

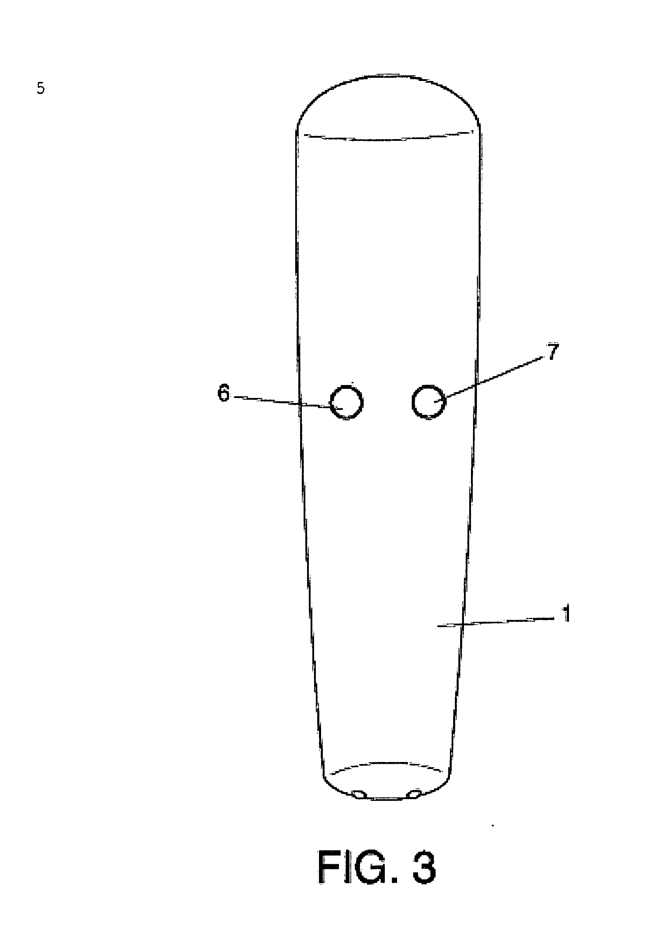

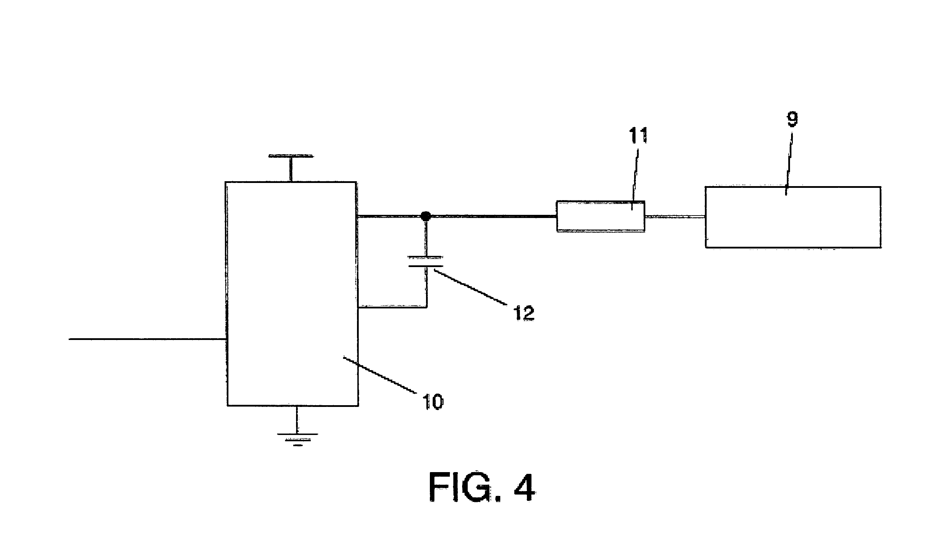

Figure 3 shows the start up and shutdown means (6, 7) of the device and the vibrator element. Said start up and shutdown means (6, 7) consists of an electrode (9), a detection chip (10) connected to the electrode (9) through a resistor (11), a capacitor (12) connected to the detection chip (10), and a logic interpretation, as shown in Figure 4.

The start up and shutdown means (6, 7) operates so that when contacting a part of the user body, the fingertip for example, to the electrode (9) causes a change of state of the start up and shutdown means (6, 7). The change will occur due to the charge variation in the capacitor (12) by the effect of the electric charge of the human body.

The values of the resistor (11) and capacitor (12) are specific of each application and must be calibrated by testing with the final materials. Said changes of state may cause that the device or vibrator element is stated up when it was off, or turned off when it was running.

**Claims**

1. Device for stimulating hair growth comprising a casing (1) with a first hold end (2) of the device, and a second stimulating end (3) comprising laser lighting means (4) and LED lighting means LED (5) as well as start up and shutdown means (6,7) of the device; wherein said laser lighting means (4) consisting of, at least, a laser lighting means (4) which emits at a wavelength between 600 and 900 nm; being said device **characterized in that** the LED lighting means (5) consisting of, at least, a LED lighting device (5) which emits at a wavelength between 400 and 600 nm; and where also includes means also includes means to turn on the laser lighting element (4) when the second stimulating end (3) is in contact with the user skin, said means consisting of in at least one contact sensor (13).

2. Device according to claim 1, wherein the contact sensor (13) is resistive or conductive.

3. Device according to claim 1 y 2, **characterized in that** the at least one laser lighting element (4) emits at a wavelength between 630 and 685 nm.

4. Device according to any of claims 1-3, **characterized in that** the at least one laser lighting element (4) emits at a wavelength equal to 655 nm.

5. Device according to any of claims 1-4, **characterized in that** the at least one LED lighting element (5) emits at a wavelength between 450 and 495 nm.

6. Device according to any of claims 1-5, **characterized in that** the at least one LED lighting element (5) emits at a wavelength equal to 470 nm.

7. Device according to any of claims 1-6, **characterized in that** comprises a vibrator element configured to vibrate the device.

8. Device according to claim 7, wherein further comprises start up and shutdown means (7) of the vibrator element.

9. Device according to any preceding claim wherein the second stimulating end (3) comprises a plurality of protrusions (8) regularly arranged configured to act like the teeth of a comb.

10. Device according to any of claims 3 o 4, **characterized in that** each laser lighting element (4) emits with a power between 3 and 10 mW.

11. Device according to claim 10, **characterized in that** each laser lighting element (4) emits with a power of 5 mW.

12. Device according to any of claims 1-11, **characterized in that** comprises more than two laser lighting elements (4), being said laser lighting elements (4) regularly distributed by the casing (1) of the second stimulating end (3).

13. Device according to any of claims 1-12, **characterized in that** the at least one laser lighting element (4) is an InGaAlP diode laser.

14. Device according to claim 8, **characterized in that** the start up and shutdown means (6) of the device and the start up and shutdown means (7) of the vibrator element comprise an electrode (9), a detection chip (10) connected to the electrode (9) through a resistor (11), a capacitor (12) connected to the detection chip (10), and an interpretation logic, so that the contact of a part of the user body to the electrode (9) causes a change of state in the start up and shutdown means (6, 7), from start up state to shutdown, or from shutdown state to start up.

FIG. 1

FIG. 2

FIG. 2A

FIG. 3

FIG. 4

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | EP 2 140 911 A1 (ACTERVIS GMBH [CH]) 6 January 2010 (2010-01-06) * paragraphs [0005], [0006], [0023] - [0027] * * figures * | 1-14 | INV. A61N5/06 |
| Y | WO 2005/046793 A2 (PALOMAR MEDICAL TECH INC [US]; ALTSHULER GREGORY B [US]; CARUSO JOSEPH) 26 May 2005 (2005-05-26) * page 25, line 28 - page 26, line 15 * | 1-14 | |
| A | WO 2007/111397 A1 (MAX ENGINEERING CO LTD [KR]; CHOI HAK-KI [KR]; HA TAE-HO [KR]; HWANG H) 4 October 2007 (2007-10-04) * paragraph [0003] * | 2,14 | |
| Y | WO 2005/115263 A1 (OSTERN CO LTD [KR]; PYUN WOONG BEOM [KR]) 8 December 2005 (2005-12-08) * paragraph [0043] * | 13 | |

TECHNICAL FIELDS
SEARCHED (IPC)

A61N

The present search report has been drawn up for all claims

| Place of search | Date of completion of the search | Examiner |
|---|---|---|
| Munich | 7 September 2011 | Lohmann, Stefan |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**ANNEX TO THE EUROPEAN SEARCH REPORT
ON EUROPEAN PATENT APPLICATION NO.**                EP 11 38 2110

This annex lists the patent family members relating to the patent documents cited in the above-mentioned European search report.
The members are as contained in the European Patent Office EDP file on
The European Patent Office is in no way liable for these particulars which are merely given for the purpose of information.

07-09-2011

| Patent document cited in search report | | Publication date | Patent family member(s) | | Publication date |
|---|---|---|---|---|---|
| EP 2140911 | A1 | 06-01-2010 | US | 2010004570 A1 | 07-01-2010 |
| WO 2005046793 | A2 | 26-05-2005 | AU | 2004289230 A1 | 26-05-2005 |
| | | | CA | 2543152 A1 | 26-05-2005 |
| | | | CN | 1901968 A | 24-01-2007 |
| | | | EP | 1697003 A2 | 06-09-2006 |
| | | | JP | 2007510466 A | 26-04-2007 |
| | | | US | 2004147984 A1 | 29-07-2004 |
| WO 2007111397 | A1 | 04-10-2007 | US | 2010042083 A1 | 18-02-2010 |
| WO 2005115263 | A1 | 08-12-2005 | CN | 1953717 A | 25-04-2007 |
| | | | EP | 1793759 A1 | 13-06-2007 |
| | | | JP | 2007537819 A | 27-12-2007 |
| | | | US | 2008269732 A1 | 30-10-2008 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 0139835 A
- US 20020128696 A
- WO 2005016454 A
- EP 2140911 A